(19) **Europäisches Patentamt European Patent Office Office européen des brevets**

(11) **EP 3 712 601 B1**

(12) **EUROPEAN PATENT SPECIFICATION**

(45) Date of publication and mention of the grant of the patent:
**19.07.2023 Bulletin 2023/29**

(21) Application number: **17932311.8**

(22) Date of filing: **15.12.2017**

(51) International Patent Classification (IPC):
**G01N 23/203** (2006.01)      **G01N 23/20** (2018.01)
**G01N 23/205** (2018.01)

(52) Cooperative Patent Classification (CPC):
**G01N 23/203;** G01N 2223/418; G01N 2223/602;
Y02E 30/30

(86) International application number:
**PCT/KR2017/014804**

(87) International publication number:
**WO 2019/098448 (23.05.2019 Gazette 2019/21)**

(54) **METHOD FOR MEASURING DEGREE OF RECRYSTALLIZATION OF ZIRCONIUM ALLOY CLADDING TUBE FOR NUCLEAR FUEL BY USING EBSD PATTERN QUALITY**

VERFAHREN ZUR MESSUNG DES REKRISTALLISATIONSGRADES VON HÜLLROHR AUS ZIRKONIUMLEGIERUNG FÜR KERNBRENNSTOFF UNTER VERWENDUNG VON EBSD-STRUKTURQUALITÄT

PROCÉDÉ DE MESURE DU DEGRÉ DE RECRISTALLISATION D'UN TUBE DE GAINAGE EN ALLIAGE DE ZIRCONIUM DESTINÉ À UN COMBUSTIBLE NUCLÉAIRE À L'AIDE D'UNE QUALITÉ DE MOTIF EBSD

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.11.2017 KR 20170154196**

(43) Date of publication of application:
**23.09.2020 Bulletin 2020/39**

(73) Proprietor: **Kepco Nuclear Fuel Co., Ltd Daejeon 34057 (KR)**

(72) Inventors:
• **JUNG, Tae Sik**
  **Daejeon 34130 (KR)**
• **MOK, Yong Kyoon**
  **Daejeon 34071 (KR)**
• **KIM, Yoon Ho**
  **Daejeon 35379 (KR)**
• **LEE, Chung Yong**
  **Daejeon 34071 (KR)**
• **JANG, Hun**
  **Sejong 30064 (KR)**

(74) Representative: **Cabinet Netter**
  **36, avenue Hoche**
  **75008 Paris (FR)**

(56) References cited:
WO-A1-2017/169837      KR-A- 20040 090 662
KR-B1- 100 660 209      KR-B1- 101 112 027
KR-B1- 101 493 944      US-B2- 9 002 499

• UNE KATSUMI ET AL: "Crystallography of Zirconium Hydrides in Recrystallized Zircaloy-2 Fuel Cladding by Electron Backscatter Diffraction", JOURNAL OF NUCLEAR SCIENCE AND TECHNOLOGY., vol. 41, no. 7, 1 July 2004 (2004-07-01), pages 731-740, XP055820440, JP ISSN: 0022-3131, DOI: 10.1080/18811248.2004.9715540
• D. DINGLEY: "Progressive steps in the development of electron backscatter diffraction and orientation imaging microscopy", JOURNAL OF MICROSCOPY, vol. 213, no. 3, 1 March 2004 (2004-03-01) , pages 214-224, XP055111021, ISSN: 0022-2720, DOI: 10.1111/j.0022-2720.2004.01321.x

• TIAN HANG ET AL: "Recrystallization behavior of cold-rolled Zr-1Nb a", JOURNAL OF NUCLEAR MATERIALS, ELSEVIER B.V, NETHERLANDS, vol. 456, 13 October 2014 (2014-10-13), pages 321-328, XP029101039, ISSN: 0022-3115, DOI: 10.1016/J.JNUCMAT.2014.09.084

**Description**

BACKGROUND OF THE INVENTION

**Technical Field**

The present invention relates generally to a method for measuring a degree of recrystallization of metal and, more particularly, to a method for measuring a degree of recrystallization of s zirconium alloy cladding tube for nuclear fuel by using EBSD pattern quality

**Background Art**

Generally, metal products used in industry are made into shapes suitable for use through final molding before being released as final products and come to have characteristics satisfying product performance requirements through final heat treatment.

Such product characteristics are directly related to a state of crystal grains forming a microstructure, and are accordingly determined by microstructural changes caused by the final heat treatment applied to the product after cold working.

Therefore, the final heat treatment in terms of producing the product is the last step capable of controlling the material characteristics of the product.

Effects of the final heat treatment affecting the product characteristics may be described in detail as follows.

The cold working of the final heat treatment macroscopically causes the deformation of the crystal grains composing the microstructure by forcing the atoms to move in a short time duration in a state of not gaining thermal assistance.

In a microscopic point of view, the generation and transfer of potential are begun for deformation in the crystal grains, and an increase of potential density due to continuous processing destroys the interatomic arrangement and eventually renders the microstructure disordered to such an extent that the crystal grains and grain boundaries may not be distinguished. A material composed of such a wrought structure is no longer capable of generating and transferring the potential, thereby having a high mechanical strength. However, improvement through heat treatment is required due to a low elongation rate.

That is, through the heat treatment, defects in the deformed crystal grains showing embrittlement characteristics are extinguished or existing crystal grains are replaced with newly formed defect-free crystal grains, whereby softening characteristics are added to the material.

These phenomena are called recovery and recrystallization, respectively, and generally the recrystallization takes place at relatively higher temperatures compared with the recovery. Out of the recovery and the recrystallization by the heat treatment determining the final product characteristics, the recrystallization is represented as a quantitative fraction called a degree of the recrystallization which is mainly used as a heat treatment target or a material characteristic parameter capable of predicting the behavior of a material.

Eventually, the fraction of the total volume of the existing recrystallized grains may be represented as the degree of the recrystallization, and this fraction is represented even as a ratio of mechanical strength. In reality, the degree of the recrystallization of materials is evaluated in the industry using two conventional methods described below.

A first method called a method for measuring a recrystallization volume fraction generally uses microstructure photographs taken by using Transmission Electron Microscopy (TEM). At this time, a fraction, Stotal/SRex, of the total two dimensional area Stotal versus the recrystallized grain area SRex is defined as the degree of the recrystallization by using a method for measuring a three-dimensional space in two dimensions in the same manner as a general crystal grain size measurement.

However, due to the nature of TEM photographs, the maximum photographable range for observing crystal grains is so narrow in units of several tens of micrometers that information provided therein is insufficient to represent the material. In addition, the criterion for determining recrystallized grain and processed crystal grain is ambiguous, whereby a result slightly different from an actual value of the degree of the recrystallization may be obtained.

A second method called a method for measuring mechanical strength is usually calculated using the yield strength. Specifically, the yield strength Ymin obtained by a uniaxial tensile test after fully recrystallizing the processed material by heat treatment is set to a 100% degree of the recrystallization, and the yield strength Ymax of the material in a state without heat treatment is set to a 0% degree of the recrystallization. Accordingly, since the yield strength Yp of the partially recrystallized material lies between 0 and 100% degrees of the recrystallization, an amount of strength decreased from Ymax relative to a difference between Ymax and Ymin is calculated as the degree of the recrystallization $X_Y$, **rex** of the material as shown in equation 1 below.

[Equation 1]

$$X_{Y,rex} = \frac{Y_{max} - Y_p}{Y_{max} - Y_{min}} \times 100, \%$$

Since the yield strength is a result due to a lot of crystal grains, the yield strength is able to represent information of the microstructure state. However, in the case of a material heat-treated at a temperature no greater than recrystallization temperature Trex, a calculation result may be caused to be far away from the actual degree of recrystallization, because the reduction in strength due to the recovery occurs dominantly rather than the reduction in strength due to formation of the recrystallized grains.

Generally, recrystallization temperature Trex of zirconium alloy cladding tube for nuclear fuel is about 500°C. As a matter of fact, papers dealing with modeling of the degree of the recrystallization through the measurement of the yield strength of zirconium alloys show that the experiment has been performed excluding the cases where zirconium alloys are heat-treated at no higher than Trex of 500°C [Hang Tian et al., J. Nucl. Mater. 456 (2015) p.321, Y. I. Jung et al., J. Alloys Comp. 497 (2009) p.423].

This is because inappropriate results are produced from modeling calculations of the degree of the recrystallization, because of a reduction in strength due to the recovery rather than a reduction in strength due to the thermally-induced recrystallization.

Since the final heat treatment temperature of the zirconium alloy cladding tube for the nuclear fuel used in nuclear power plants is no greater than 500°C, nuclear fuel manufacturers use TEM photographs to measure the degree of the recrystallization of zirconium alloy cladding tube. In general, a TEM photograph allows only local areas in the range of up to tens of square microns to be observed, whereby a number of multiple TEM photographing are required to demonstrate the typical degree of the recrystallization of heat-treated samples.

In Korean Patent No. 10-1,493,944, after selecting a critical value by measuring an intragranular orientation spread value of metal material by using EBSD, the degree of the recrystallization is measured through a process comparing the critical value with intragranular orientation spread values of the crystal grains of no less than a specific size.

In U.S. Patent No. 9,002,499, a method is proposed to determine a degree of the recovery by measuring azimuth angle of crystal grains of metal material by using the EBSD.

UNE KATSUMI ET AL ("Crystallography of Zirconium Hydrides in Recrystallized Zircaloy-2 Fuel Cladding by Electron Backscatter Diffraction",JOURNAL OF NUCLEAR SCIENCE AND TECHNOLOGY., vol. 41, no. 7, 1 July 2004 (2004-07-01), pages 731-740) discloses a method of analysing the recrystallization of zirconium alloys for nuclear fuel by Electron Backscatter Diffraction.

WO 2017/169837 A1 discloses a method of characterizing rolled steel by the properties of the histogram of EBSD pattern quality.

Up to now, the method for measuring the degree of the recrystallization by using the EBSD, like the existing registered patents, has stated various analytical methods capable of determining whether a crystal grain is recrystallized or not according to a difference of azimuth angles after measuring crystallographic orientations of the crystal grains. In addition, much literature has also reported methods for determining recrystallized grains on the basis of crystallographic orientation.

A problem associated with such methods is that in order to determine whether a measured crystal grain is recrystallized or not, it is necessary to set the critical intragranular orientation spread value and minimum effective crystal grain size as references through a basic experiment. In other words, when the two critical values above are set incorrectly, there is a possibility to get values different from the actual degree of the recrystallization of the material.

Therefore, a technique that may identify with a degree of high accuracy whether the recrystallization rather than the recovery has occurred and to what degree the recrystallization has progressed, without passing through precise measurements which entail numerous trials and errors is needed.

Documents of Related Art

(Patent Document 1) Korean Patent No. 10-1493944 (Date of Patent: Feb. 10, 2015)
(Patent Document 2) US Patent No. 9,002,499 (Date of Patent: Apr. 7, 2015)

## Disclosure

### Technical Problem

Accordingly, the present invention has been made keeping in mind the above problems occurring in the related art, and the present invention is intended to provide a more stable and accurate method for calculating a degree of recrystallization of a zirconium alloy cladding tube for nuclear fuel in various temperature ranges, by using pattern quality values measured by utilizing EBSD, compared with an existing calculation method.

### Technical solution

In order to achieve the above objective, according to one aspect of the present invention, there is provided a method for measuring a degree of recrystallization of a zirconium alloy cladding tube, the method including: electrolytic polishing a fully recrystallized sample 1, a partially recrystallized sample 2 of which a recrystallization measurement is needed, and an as-deformed sample 3, then making an SEM electron beam incident to each of the sample 1 to the sample 3 at a predetermined scan interval, wherein each sample is zirconium alloy, and then acquiring a backscattered electron signal through an EBSD camera (step 1); using EBSD software so as to convert the backscattered electron signals acquired from the sample 1 to the sample 3 into pattern quality values, and calculating the pattern quality values as frequencies within a specific range (step 2); obtaining a pattern quality frequency (B + D) deviated from a frequency of the sample 3 among a whole frequency distribution of the sample 2, and obtaining a pattern quality frequency (D + E) deviated from the frequency of the sample 3 among a total frequency distribution of the sample 1 (step 3); and a fourth step of obtaining a degree of recrystallization of the sample 2 from equation $X' = \frac{(B+D)}{(D+E)} \times 100$, % (step 4).

Here, an area which SEM beams may be made incident to each of the sample 1 to the sample 3 in step 1 is 100 $\mu$m $\times$ 100 $\mu$m;

In addition, the predetermined scan interval in step 1 may be 200 to 300 nm.

In addition, the range of the pattern quality values may be step 2 is 100 to 1000.

### Advantageous Effects

A method, for calculating a degree of recrystallization of a zirconium cladding tube for nuclear fuel by using EBSD according to the present invention, may reinforce against both of the inaccurate calculation of a degree of recrystallization using mechanical strength caused by the strength reduction due to recovery when heat-treated at no greater than the recrystallization temperature, and the possibility of obtaining insufficient recrystallization information due to observation of local areas when TEM photographs are utilized. In addition, a more accurate degree of the recrystallization can be obtained compared with a method for calculating the degree of the recrystallization by using existing pattern quality.

### Description of Drawings

FIG. 1 is a histogram illustrating a frequency after arbitrarily categorizing pattern quality of an heat-treated zirconium cladding tube into specified ranges,

FIG. 2 illustrates schematic line graphs showing cumulative frequency, wherein areas enclosed by the line graphs are divided into A, B, C, D, and E,

FIG. 3a shows three-dimensional histograms illustrating distribution charts of pattern quality of a zirconium cladding tube heat-treated at various temperatures measured through an embodiment,

FIG. 3b shows two-dimensional line graphs illustrating distribution charts of the pattern quality of the zirconium cladding tube heat-treated at various temperatures measured through the embodiment,

FIG. 4 illustrates line graphs for values of degrees of recrystallization obtained using equations 1 to 3,

FIG. 5 illustrates photographs of crystal grains of the embodiment observed at a magnification of 4,900 times by using a TEM, and

FIG. 6 illustrates line graphs representing the pattern quality distribution charts of a sample at 520, 540, and 560°C, respectively, wherein each line graph is shown taking line graphs of as-deformed and fully recrystallized samples as references.

Best Mode

Specific structures or functional descriptions presented in an embodiment of the present invention are merely illustrative for the purpose of describing the embodiment according to the concept of the present invention, and embodiments according to the concept of the present invention may be implemented in various forms. In addition, the present invention should not be construed as limited to the embodiments set forth herein, but should be understood to include all modifications, equivalents, and alternatives falling within the spirit and scope thereof.

Hereinbelow, the present invention will be described in detail with reference to the accompanying drawings.

As described in a section of the Background Art above, there are difficult aspects to precisely and stably calculate a degree of recrystallization using a method for measuring the degree of the recrystallization through setting of critical values of both an azimuth angle of the crystal grains and a minimum crystal grain size.

Accordingly, a value called pattern quality obtainable from EBSD is introduced in a calculation of the degree of the recrystallization in the present invention.

The pattern quality represents a value of a degree of sharpness of a Kikuchi line that is an electron beam of a Scanning Electron Microscopy (represented as SEM hereafter) coming out by backscattered diffraction from a sample after entering the sample. Meanwhile, the degree of the recrystallization may be calculated by comparing a value of the pattern quality of a partially crystallized material with values of the pattern quality of a fully recrystallized sample and an as-deformed sample.

A method for calculating the degree of the recrystallization through obtaining the value of the pattern quality has been introduced for only copper alloy in the existing literature [Tarasiuk et al., Acta Mater. 50 (2002) p.1467]. However, there is a problem that the corresponding calculation method may not obtain an accurate degree of recrystallization from the pattern quality distribution of the zirconium cladding tube for nuclear fuel.

Therefore, it is necessary to improve the method for calculating the degree of the recrystallization by using the pattern quality.

Accordingly, a method for calculation of the present invention will be described as below and validity thereof will be verified through an appropriate example.

Characteristics of the EBSD provided in the SEM used in the present invention is as follows.

Arrangement of atoms composing material is positioned in a different direction for each crystal grain, and a short wavelength electron entered into the material from an electron gun of the SEM comes out by diffraction from each of the crystal grains, wherein the electron is recognized as a Kikuchi line by an EBSD detector. At this time, the Kikuchi line is stored as a value of the pattern quality representing a degree of sharpness through an independent calculation at the inside of the detector.

A sharp Kikuchi line implies the arrangement of atoms is extremely regular and indicates a corresponding measuring part has no defect by being recrystallized, thereby having a high pattern quality index. On the contrary, since the arrangement of atoms of a wrought structure is disorderedly distributed and has many defects, it is difficult for the entered electron to be diffracted, whereby a blurred Kikuchi line appears resulting in obtaining a low value of the pattern quality.

Prior to describing a detailed method for calculating the degree of the recrystallization characterizing the invention, a method for obtaining a value of the pattern quality used as a calculation input value and obtained from the EBSD may be described as follows.

The value of the pattern quality is a numerical result obtained by shooting an electron beam in the manner of scanning while moving by a preset interval within a specified area of a material surface being observed by the SEM. The value of the pattern quality contains information of the material surface scanned in the designated area and the degree of the recrystallization is calculated with these values as input values.

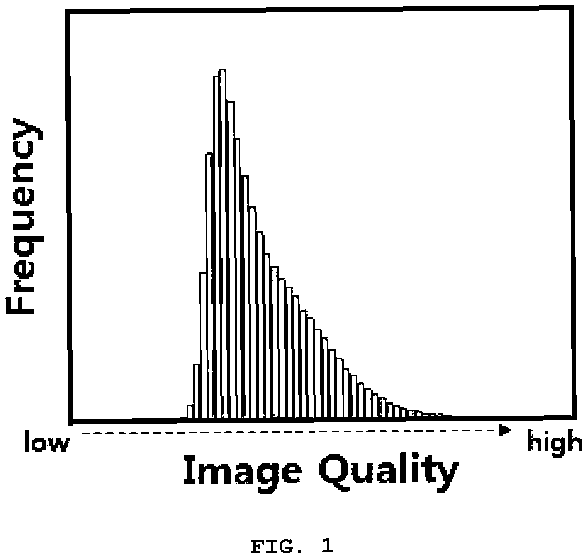

FIG. 1 is a histogram illustrating a distribution of the values of each pattern quality after measuring the frequency by the corresponding range after categorizing, by arbitrary range, actual values of the pattern quality obtained from arbitrary material.

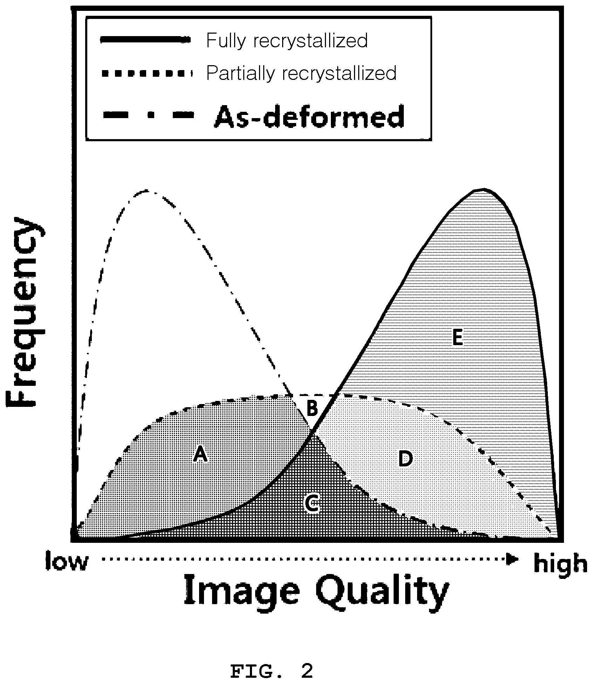

In FIG. 2, each of trends of frequency distributions having different degrees of the recrystallization of arbitrary material is illustrated in schematic line graphs by connecting a maximum point at each pattern quality value to each other, wherein areas overlapping each other or separated from each other are indicated as A, B, C, D, and E.

As the material is heat-treated more closely to be fully recrystallized as shown in FIG. 2, frequency values of the pattern quality of the material are distributed skewed toward higher pattern quality value.

Tarasiuk calculated a degree of the recrystallization X by taking the pattern quality as an input value using equation 2 below:

[Equation 2]

$$X = \left\{ \left( \frac{B+D}{\text{Total measured frequency}} \right) \times \left[ 1 + \left( \frac{C-B}{\text{Total measured frequency}} \right) \right] \right\} \times 100, \%$$

Here, as shown in FIG. 2, B represents a sum of frequencies of an area where the frequency of the pattern quality distribution of the partially recrystallized sample is greater than that of the as-deformed sample or the fully recrystallized sample; C represents a sum of frequencies of an area where three distributions overlap each other; D represents a sum of frequencies of an area where the frequency of the pattern quality distribution of the partially recrystallized sample is smaller than that of the fully recrystallized sample and greater than that of the as-deformed sample; E represents a sum of frequencies of an area where the frequency of the fully recrystallized sample is greater than that of the partially recrystallized sample. Meanwhile, a total measured frequency is a sum of all of A, B, C, and D.

First, step 1 of the present invention is a step in which values of the pattern quality are obtained through the EBSD using electrolytic polishing for the zirconium cladding tube, which is to be measured for a degree of the recrystallization, of the nuclear fuel. The cladding tubes used at this time were heat-treated for eight hours at 440, 460, 480, 500, 520, 540, 560, or 580°C in a vacuum state, and an as-deformed sample is measured together when the EBSD is measured. At this time, a sample heat-treated for eight hours at 580°C may be utilized as a fully recrystallized reference sample.

After heat-treatment, a surface perpendicular to an axial direction of the cladding tube was mechanically polished, and a shiny surface was additionally made through electrolytic polishing. While scanning was proceeded at 250 nm intervals for a part of 100 $\mu$m (width) $\times$ 100 $\mu$m (length) on the surface, 185,031 electron backscattered signals per a sample were received.

In step 2 of the present invention, after the measured electron backscattered signals of each of the samples are converted into values of the pattern quality by using an EBSD software, the values of the pattern quality are categorized by an arbitrarily specified interval and are cumulatively generated as frequency by the specified interval. At this time, a maximum pattern quality value was 69,647, and the frequency of each corresponding interval was generated by dividing pattern quality values of each of samples by 1,000.

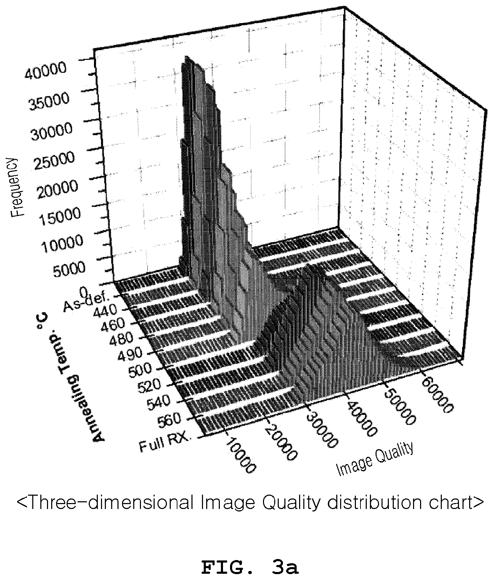

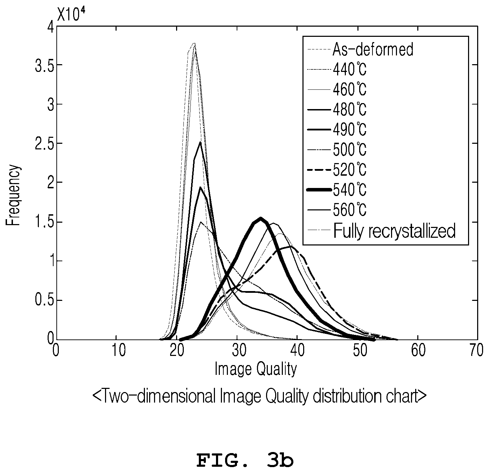

In FIGS. 3a and 3b, values of the pattern quality obtained at all heat treatment temperatures are divided into intervals by 1,000 and cumulative frequency numbers obtained at the corresponding intervals are presented in a three-dimensional histogram and two-dimensional line graphs.

In step 3 of the present invention, frequencies corresponding to areas of B, D, and E in FIG. 2 are obtained, respectively, from the as-deformed, partially recrystallized, and fully recrystallized pattern quality distribution charts.

In step 4 of the present invention, frequency calculation is performed for areas of B, D, and E obtained respectively and is as equation 3 below.

[Equation 3]

$$X' = \frac{(B+D)}{(D+E)} \times 100, \%$$

[Table 1]

| Heat treatment temperature, °C / Calculation method | 440 | 460 | 480 | 490 | 500 | 520 | 540 | 560 |
|---|---|---|---|---|---|---|---|---|
| $X_{Y, rex}$ (Yield strength, Equation 1) | 45.6% | 51.9% | 61.4% | 75.9% | 84.3% | 94.7% | 94.8% | 96.4% |
| X (Tarasiuk 1st, Equation 2) | 11.4% | 14.0% | 36.2% | 48.1% | 52.0% | 92.1% | 76.9% | 91.3% |
| X' (Present invention, Equation 3) | 12.5% | 15.9% | 41.1% | 53.7% | 59.7% | 97.4% | 95.2% | 99.3% |

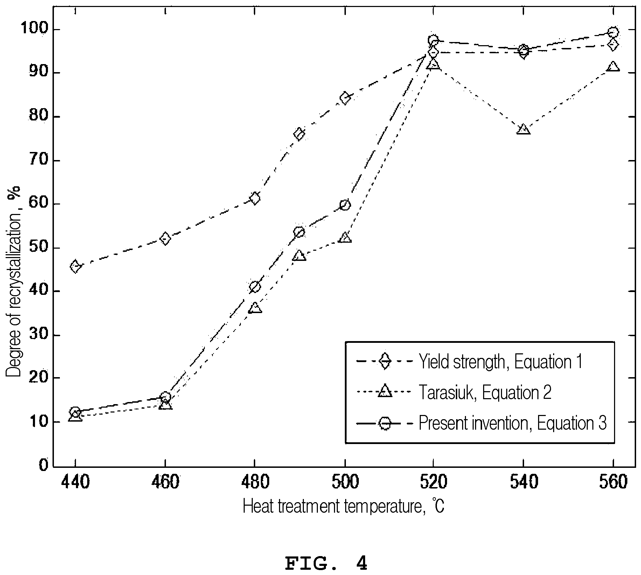

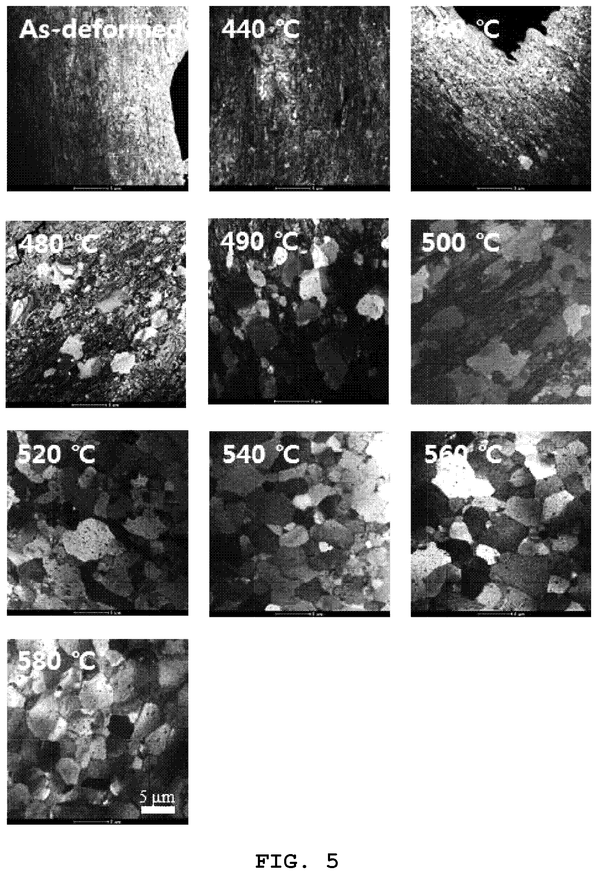

Table 1 illustrates values of the degrees of the recrystallization obtained by the calculation method of the present invention (equation 3), the method introduced in the literature of Tarasiuk (equation 2), and the calculation method through yield strength measurement value described in the section of the Background Art together, and these values of the degrees of the recrystallization are illustrated by heat treatment temperature in the line graphs of FIG. 4. In addition, FIG. 5 illustrates TEM photographs of the measured samples.

Values of the degrees of the recrystallization inferred through the microstructure photographs of FIG. 5 are shown to have a large deviation from the values of the degrees of the recrystallization $X_{Y,rex}$ calculated from the yield strength in the case of the specimen/sample heat-treated below 520°C. However, the values of the degrees of the recrystallization $X_{Y,rex}$ at no less than 520°C accord well with the values of the degrees of the recrystallization inferred through the microstructure photographs.

In FIG. 4, the values of the degrees of the recrystallization are shown to rise with increasing heat treatment temperature in line graphs, wherein the values of the degrees of the recrystallization obtained by Tarasiuk equation (equation 2) and the present invention equation (equation 3), respectively, vary in an almost same trend at each temperature. However, at 540°C, the result of equation 2 is 76.9% which is approximately 18% lower than the results of equations 2 and 3 which are 94.8 and 95.2%, respectively.

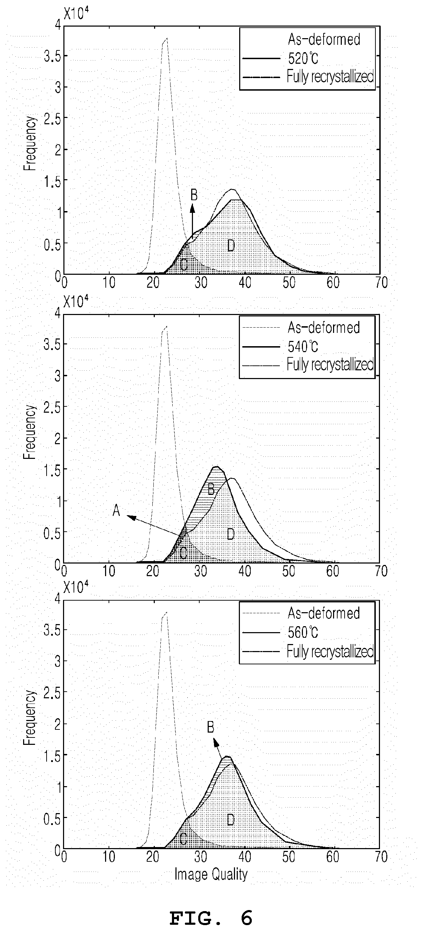

This tendency is not observed in the cases of 520°C and 560°C, and a reason therefor may be described by comparing quality value distribution charts at 520, 540, and 560°C of FIG. 6. In FIG. 6, a graph at 540°C shows a larger frequency B and a smaller frequency D compared with a graph at 520°C or 560°C. This phenomenon is arisen from nonhomogeneous recrystallization behavior of the zirconium alloy because abnormally grown crystal grains emitting a signal for area D emit a signal for area B while being recrystallized in small crystal grains at a higher temperature [G. Kumar et al., J. Nucl. Mater. 466(2015)]. That is, although crystal grains emitting a signal for area B are also crystal grains of a recrystallized area, it is set to calculate the degree of the recrystallization by lowering the contribution of the area B to the recrystallization in the calculation method of Tarasiuk.

Specifically, in the calculation method of Tarasiuk, the right term is reduced due to a high value of B, thereby reducing a value of the degree of the recrystallization. That is, the reduced value of the degree of the recrystallization is obtained at 540°C because it is not calculated for the area B being weighted the same as the area D in the relevant equation, though the area B is actually recrystallized. Since the TEM photograph at 540°C of FIG. 5 is the result to be surely judged that recrystallization fraction is at least 90%, the calculation method of Tarasiuk does not seem to be a valid method for the zirconium alloy cladding tube.

Accordingly, area B should be used with the same weighting as the area D in the calculation of a degree of the recrystallization for the zirconium alloy cladding tube. Accordingly, an improved calculation equation is suggested as equation 3.

Through the equation of the present invention, a degree of the recrystallization which is the ratio expressed as a percentage is calculated by taking the fully recrystallized pattern quality frequency (D + E) deviated from the as-deformed pattern quality distribution as a denominator and the frequency (B + D) which is the partially recrystallized pattern quality frequency deviated from the as-deformed pattern quality distribution as a nominator.

The equation of the present invention may calculate
an accurate degree of the recrystallization in a whole of temperature intervals without causing a reduction of a value of the degree of the recrystallization in a specific temperature interval for the degree of the recrystallization X' of zirconium alloy cladding tube for the nuclear fuel measured through an embodiment.

The present invention described above is to be understood not to be limited to the aforementioned embodiment and accompanying drawings. Accordingly, it will be apparent to those having ordinary knowledge and skill in the art to which the present invention pertains that various replacements, modifications, and variations are possible without departing from the scope of the appended claims.

**Claims**

1. A method for measuring a degree of recrystallization of a zirconium alloy cladding tube for nuclear fuel, the method comprising:

   electrolytic polishing a fully recrystallized sample 1, a partially recrystallized sample 2 of which a recrystallization measurement is needed, and an as-deformed sample 3, then making an SEM electron beam incident onto each of the sample 1 to the sample 3 at a predetermined scan interval, and then acquiring a backscattered electron signal through an EBSD camera of each of the sample 1 to the sample 3 (step 1);
   using EBSD software so as to convert the backscattered electron signals acquired from each of the sample 1 to the sample 3 into pattern quality values, and calculating pattern quality values histograms as frequencies by a specific range (step 2);
   obtaining pattern quality frequencies B + D deviated from the pattern quality values histograms of the sample 3 among the pattern quality values histograms of the sample 2, B representing a sum of frequencies of an area where the frequency of the pattern quality distribution of the partially recrystallized sample 2 is greater than that of the as-deformed sample 3 and the fully recrystallized sample 1, D representing a sum of frequencies of an area where the frequency of the pattern quality distribution of the partially recrystallized sample 2 is smaller than that of the fully recrystallized sample 1 and greater than that of the as-deformed sample 3, and obtaining pattern quality frequencies D + E deviated from the pattern quality values histograms of the sample 3 among the pattern quality values histograms of the sample 1, E representing a sum of frequencies of an area where the frequency of the fully recrystallized sample 3 is greater than that of the partially recrystallized sample 1 (step 3); and

obtaining a degree of recrystallization of the sample 2 from equation $X' = \frac{(B+D)}{(D+E)} \times 100$, % (step 4).

2. The method of claim 1, wherein an area which SEM beams are made incident to each of the sample 1 to the sample 3 in step 1 is 100 $\mu$m $\times$ 100 $\mu$m;

3. The method of claim 1, wherein the predetermined scan interval in step 1 is 200 to 300 nm.

4. The method of claim 1, wherein the specific range of the pattern quality values in step 2 is 100 to 1000.

**Patentansprüche**

1. Verfahren zur Messung des Rekristallisationsgrades eines Hüllrohrs aus einer Zirkoniumlegierung für Kernbrennstoff, wobei das Verfahren umfasst:

elektrolytisches Polieren einer vollständig rekristallisierten Probe 1, einer teilweise rekristallisierten Probe 2, für die eine Rekristallisationsmessung erforderlich ist, und einer unverformten Probe 3, anschließendes Auftreffenlassen eines SEM-Elektronenstrahls auf jede der Proben 1 bis 3 in einem vorbestimmten Abtastintervall und anschließendes Erfassen eines Signals rückgestreuter Elektronen durch eine EBSD-Kamera von jeder der Proben 1 bis 3 (Schritt 1);
Verwenden einer EBSD-Software derart, dass die Signale der rückgestreuten Elektronen, die von jeder der Probe 1 bis zur Probe 3 erfasst wurden, in Musterqualitätswerte umgewandelt werden, und Berechnen von Histogrammen von Musterqualitätswerten als Frequenzen in einem bestimmten Bereich (Schritt 2);
Ermitteln von Musterqualitätsfrequenzen B + D, die von den Histogrammen der Musterqualitätswerte der Probe 3 unter den Histogrammen der Musterqualitätswerte der Probe 2 abweichen, wobei B eine Summe von Frequenzen eines Bereichs darstellt, in dem die Frequenz der Musterqualitätsverteilung der teilweise rekristallisierten Probe 2 größer ist als die der unverformten Probe 3 und der vollständig rekristallisierten Probe 1, D eine Summe von Frequenzen eines Bereichs darstellt, in dem die Frequenz der Musterqualitätsverteilung der teilweise rekristallisierten Probe 2 kleiner ist als die der vollständig rekristallisierten Probe 1 und größer als die der unverformten Probe 3, und Ermitteln von Musterqualitätsfrequenzen D + E, die von den Histogrammen der Musterqualitätswerte der Probe 3 unter den Histogrammen der Musterqualitätswerte der Probe 1 abweichen, wobei E eine Summe von Frequenzen eines Bereichs darstellt, in dem die Frequenz der vollständig rekristallisierten Probe 3 größer ist als die der teilweise rekristallisierten Probe 1 (Schritt 3); und

Ermitteln eines Rekristallisationsgrads der Probe 2 aus der Gleichung $X' = \frac{(B+D)}{(D+E)} x 100$, % (Schritt 4).

2. Verfahren nach Anspruch 1, wobei der Bereich, in dem die SEM-Strahlen in Schritt 1 jeweils auf die Probe 1 bis die Probe 3 treffen, 100 $\mu$m $\times$ 100 $\mu$m beträgt;

3. Verfahren nach Anspruch 1, wobei das vorbestimmte Scanintervall in Schritt 1 200 bis 300 nm beträgt.

4. Verfahren nach Anspruch 1, wobei der spezifische Bereich der Musterqualitätswerte in Schritt 2 100 bis 1000 beträgt.

**Revendications**

1. Procédé pour mesurer un degré de recristallisation d'un tube de gainage en alliage de zirconium pour combustible nucléaire, le procédé comprenant :

polir par voie électrolytique un échantillon 1 entièrement recristallisé, un échantillon 2 partiellement recristallisé dont une mesure de recristallisation est nécessaire, et un échantillon 3 tel que déformé, puis rendre un faisceau d'électrons de microscope électronique à balayage, SEM, incident sur chacun de l'échantillon 1 à l'échantillon 3 à un intervalle de balayage prédéterminé, puis l'acquisition d'un signal d'électrons rétrodiffusés par l'intermédiaire d'une caméra EBSD de chacun de l'échantillon 1 à l'échantillon 3 (étape 1) ;

utiliser un logiciel EBSD de manière à convertir les signaux d'électrons rétrodiffusés acquis depuis chacun de l'échantillon 1 à l'échantillon 3 en valeurs de qualité de motif, et calculer des histogrammes de valeurs de qualité de motif comme fréquences par une plage spécifique (étape 2) ;

obtenir des fréquences de qualité de motif B + D déviées des histogrammes de valeurs de qualité de motif de l'échantillon 3 parmi les histogrammes de valeurs de qualité de motif de l'échantillon 2, B représentant une somme de fréquences d'une aire où la fréquence de la répartition de qualité de motif de l'échantillon 2 partiellement recristallisé est supérieure à celle de l'échantillon 3 tel que déformé et de l'échantillon 1 entièrement recristallisé, D représentant une somme de fréquences d'une aire où la fréquence de la répartition de qualité de motif de l'échantillon 2 partiellement recristallisé est inférieure à celle de l'échantillon 1 entièrement recristallisé et supérieure à celle de l'échantillon 3 tel que déformé, et obtenir des fréquences de qualité de motif D + E déviées des histogrammes de valeurs de qualité de motif de l'échantillon 3 parmi les histogrammes de valeurs de qualité de motif de l'échantillon 1, E représentant une somme de fréquences d'une aire où la fréquence de l'échantillon 3 entièrement recristallisé est supérieure à celle de l'échantillon 1 partiellement recristallisé (étape 3) ; et

obtenir un degré de recristallisation de l'échantillon 2 à partir de l'équation $X' = \frac{(B+D)}{(D+E)} \times 100$, % (étape 4).

2. Procédé selon la revendication 1, dans lequel une aire dans laquelle des faisceaux SEM sont rendus incidents à chacun de l'échantillon 1 à l'échantillon 3 à l'étape 1 est 100 $\mu$m $\times$ 100 $\mu$m.

3. Procédé selon la revendication 1, dans lequel l'intervalle de balayage prédéterminé à l'étape 1 est de 200 à 300 nm.

4. Procédé selon la revendication 1, dans lequel la plage spécifique des valeurs de qualité de motif à l'étape 2 est de 100 à 1000.

FIG. 1

FIG. 2

<Three-dimensional Image Quality distribution chart>

FIG. 3a

<Two-dimensional Image Quality distribution chart>

FIG. 3b

FIG. 4

FIG. 5

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 101493944
- US 9002499 B
- WO 2017169837 A1

### Non-patent literature cited in the description

- **HANG TIAN et al.** *J. Nucl. Mater.,* 2015, vol. 456, 321
- **Y. I. JUNG et al.** *J. Alloys Comp.,* 2009, vol. 497, 423
- **UNE KATSUMI et al.** Crystallography of Zirconium Hydrides in Recrystallized Zircaloy-2 Fuel Cladding by Electron Backscatter Diffraction. *JOURNAL OF NUCLEAR SCIENCE AND TECHNOLOGY.,* 01 July 2004, vol. 41 (7), 731-740
- **TARASIUK et al.** *Acta Mater.,* 2002, vol. 50, 1467
- **G. KUMAR et al.** *J. Nucl. Mater.,* 2015, 466